(19) Europäisches Patentamt
European Patent Office
Office européen des brevets

(11) EP 2 080 526 A1

(12) **EUROPEAN PATENT APPLICATION**
published in accordance with Art. 153(4) EPC

(43) Date of publication:
**22.07.2009 Bulletin 2009/30**

(21) Application number: **07806515.8**

(22) Date of filing: **31.08.2007**

(51) Int Cl.:
***A61K 51/00*** *(2006.01)* ***A61K 47/22*** *(2006.01)*
***G01T 1/161*** *(2006.01)*

(86) International application number:
**PCT/JP2007/067042**

(87) International publication number:
**WO 2008/056481 (15.05.2008 Gazette 2008/20)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **09.11.2006 JP 2006304338**

(71) Applicant: **NIHON MEDI-PHYSICS CO., LTD.**
**Tokyo 136-0075 (JP)**

(72) Inventors:
- **HAYASHI, Akio**
**Sodegaura-shi**
**Chiba 299-0266 (JP)**
- **KUROSAKI, Fumie**
**Sodegaura-shi**
**Chiba 299-0266 (JP)**

(74) Representative: **Wilhelms · Kilian & Partner**
**Patentanwälte**
**Eduard-Schmid-Strasse 2**
**81541 München (DE)**

(54) **RADIOACTIVE DIAGNOSTIC IMAGING AGENT**

(57) A composition comprising a radioactive fluorine-labeled amino acid compound, which is reduced in radiolysis, is provided. A composition is produced by mixing the radioactive fluorine-labeled amino acid compound with a sugar lactone in an amount effective to prevent the radiolysis. As the sugar lactone, one selected from the group consisting of ascorbic acid and glucono-σ-lactone can preferably be used. The sugar lactone is preferably one selected from the compounds that are acceptable as pharmaceutical additives. The composition preferably contains the sugar lactone in an amount not less than 0.5 μmol/mL and not more than an amount acceptable for pharmaceutical additives. Preferably, the composition is prepared to have a radioactive concentration in use of 25-125 MBq/mL.

EP 2 080 526 A1

## Description

TECHNICAL FIELD

The present invention relates to a composition of a radioactive fluorine-labeled amino acid compound. More specifically, it relates to a composition of a radioactive fluorine-labeled amino acid compound useful for detecting tumors by positron emission tomography.

BACKGROUND ART

Nuclear medicine examination enables diagnosis by administering an agent containing a compound labeled with a specific radioisotope as an effective ingredient (hereinafter referred to as "radiopharmaceutical") intravenously to a human body and detecting a radiation emitted from the compound, followed by imaging based on information obtained from the radiation. Nuclear medicine examination is effective in diagnosing a variety of diseases including heart disease and cancer, and characteristic in that it has not only high specificity and sensitivity to diseases, but also an advantage of providing information on the functionality of lesions, compared to other examination techniques.

In such nuclear medicine examination, different diagnostic apparatuses require different nuclides for radiopharmaceuticals to be administered. Single photon emission computed tomography (hereinafter referred to as SPECT) is used for detention of γ rays such as $^{99m}Tc$, $^{123}I$, and $^{201}Tl$, and positron emission tomography (hereinafter referred to as PET) is used for detection of positron annihilation radiation such as $^{11}C$, $^{13}N$ .and $^{18}F$. Most of the agents used for the SPECT examination (hereinafter referred to as SPECT agents) are supplied from pharmaceutical manufacturers. In contrast, since most of the agents used for PET examination (hereinafter referred to as PET agents) contain nuclides shorter in half-life than SPECT agents, PET agents have not conventionally been supplied from pharmaceutical manufacturers, but have been prepared for use in diagnosis by an apparatus called cyclotron in hospitals. However, delivery of PET agents from pharmaceutical manufacturers has recently been started, and PET examination has become possible even in medical institutions having no cyclotron, thanks to changes in surrounding environments such as developments of manufacturing techniques and traffic networks.

Research and development taking the delivery intro account is accelerated not only for SPECT agents but also PET agents due to the above-mentioned changes in the surrounding environments. For example, amino acid compounds including [$^{18}F$]1-amino-3-fluorocyclobuthanecarboxylic acid (hereinafter referred to as anti- [$^{18}F$]-FACBC) and radioactive fluorine-labeled bodies of nucleic acid compounds such as [$^{18}F$]-fluorothymidine are synthesized to conduct research and development for PET agents. In radiopharmaceuticals such as PET agents, a problem often arises such that compounds decompose by self-radiation during delivery of the agents so as to cause decrease in radiochemical purity due to so-called radiolysis. Particularly in PET agents, radiolysis often becomes more problematic since the half-life of nuclides used therein is shorter than that of nuclides used in SPECT agents, and thus radioactivity upon shipment must be set larger than SPECT agents, thereby making the resulting radiation energy thereof higher.

For general pharmaceuticals, it is recommended in the guideline of ICH that if decomposed matters in an agent exceed 1.0%, the decomposed matters be subjected to structure determination when the maximum daily dosage of an effective component thereof is as small as not more than 1 mg (Non-Patent Document 1). In most cases, the physical amount of impurities resulting from the radiolysis which may be considered to be one of the decomposition in an agent is as small as about $10^{-12}$ mol. Thus, structure determination of the decomposed compounds by NMR analysis is difficult even though their fragments can be just presumed by mass spectrometry which is excellent in detection sensitivity. Consequently, it is very difficult to conduct verification as to whether or not the decomposed matters affect effectiveness such as tumor accumulation of the agent. Therefore, radiolysis in the radioactive diagnostic imaging agent should be maintained as low as possible.

As for a series of radioactive fluorine-labeled amino acid compounds such as the above-mentioned anti-[$^{18}F$]-FACBC, it is also requisite to reduce radiolysis thereof. In fact, various methods for preventing radiolysis have been disclosed for [$^{18}F$]-fluorodeoxyglucose (hereinafter referred to as [$^{18}F$]-FDG) which is a PET agent covered by health insurance and widely used in clinical application.

International Publication No. WO03/090789 pamphlet discloses a method of reducing the radiolysis of [$^{18}F$]-FDG by adding a weak acid-based buffer to a [$^{18}F$]-FDG solution and an injection prepared buy the method (Patent Document 1). Also, International Publication No. WO04/043497 pamphlet discloses adding ethanol to a [$^{18}F$]-FDG solution to obtain a composition of injection which may be reduced in radiolysis of [$^{18}F$]-FDG and improves in stability (Patent Document 2).

Japanese Patent Laid-open (Kokai) No. H10-147542 discloses a technique utilizing an organic compound high in physiological acceptability such as monosaccharides, disaccharides, organic acids and salts or esters thereof as a radiation protecting agent (Patent Document 3). Further, in this publication, the organic compound high in physiological acceptability is defined to have a reaction rate constant with OH radicals, H radicals or hydrated electrons in the range of $1 \times 10^8$ to $5 \times 10^{10}$ $mol^{-1}s^{-1}$.

Non-Patent Document 1: Pharmaceutical Affairs Bureau Notification No. 0624001 (page 12)
Patent Document 1: International Publication No. WO03/090789 pamphlet
Patent Document 2: International Publication No. WO04/043497 pamphlet
Patent Document 3: Japanese Patent Laid-open (Kokai) No. H10-147542

## DISCLOSURE OF THE INVENTION

## PROBLEMS TO BE SOLVED BY THE INVENTION

As described above, International Publication No. WO03/090789 pamphlet and International Publication No. WO04/043497 pamphlet disclose conditions for preventing radiolysis of [$^{18}$F]-FDG in the solution. However, these documents disclose techniques for reducing radiolysis of [$^{18}$F] -FDG only, but do not disclose any technique for reducing radiolysis of a series of radioactive fluorine-labeled amino acid compounds.
Japanese Patent Laid-open (Kokai) No. H10-147542 discloses a technique utilizing an organic compound high in physiological acceptability as a radiation protecting agent for pharmaceuticals. However, it is not apparent which compound is selected as the organic compound high in physiological acceptability or how much the compound is added in order to prevent radiolysis of the series of radioactive fluorine-labeled amino acid compounds such as anti-[$^{18}$F]-FACBC.
The present invention has been made in view of the above circumstances, and aimed at providing a composition comprising a radioactive fluorine-labeled amino acid compound, which is reduced in radiolysis.

## MEAN FOR SOLVING THE PROBLEM

As a result of diligent researches, the present inventors have found that the radiolysis can be inhibited by adding a sugar lactone to radioactive fluorine-labeled amino acid compounds, and further have found conditions suitable for inhibiting the radiolysis of radioactive fluorine-labeled amino acid compounds.
The radioactive diagnostic imaging agent according to the present invention is characterized in that it comprises a sugar lactone and a radioactive fluorine-labeled amino acid compound represented by the following formula (1) :
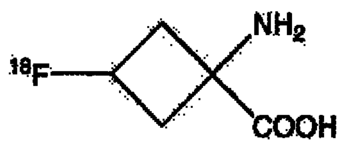

(1)

The sugar lactone used in the present invention is preferably selected from compounds acceptable as pharmaceutical additives.
As the sugar lactone, one which is selected from the group consisting of ascorbic acid and glucono-o-lactone can be used preferably. Addition amount thereof needs to be enough to obtain radiolysis inhibition effect, and is preferably not less that 0.5 μmol/mL, further preferably not less than 1.0 μmol/mL, furthermore preferably not less than 5.0 μmol/mL, and particularly preferably not less than 10 μmol/mL. The upper limit of the addition amount needs to be an amount that is acceptable for pharmaceutical additives; for examples, 2.8 g is the upper limit as a total daily dose for ascorbic acid.
In the radioactive diagnostic imaging agent according to the present invention, radioactive concentration is not particularly limited as long as a sufficient amount of radioactivity can be ensured when used. More specifically, the radioactive concentration in use is preferably 25-125 MBq/mL, and more preferably 25-100 MBq/mL.
In the present specification, compounds acceptable as pharmaceutical additives mean compounds that are approved as pharmaceutical additives in the Japanese Pharmacopoeia, the United States Pharmacopoeia, the European Pharmacopoeia, and so on. In addition, sugar lactone means a cyclic ester compound that is derived by oxidation of a sugar.

## EFFECTS OF THE INVENTION

According to the present invention, a composition of a radioactive fluorine-labeled amino acid compound which can be inhibited in radiolysis is provided.

BEST MODE FOR CARRYING OUT THE INVENTION

Hereinafter, the most preferable embodiments for the composition of the radioactive fluorine-labeled amino acid compound according to the present invention will be described.

For production of the radioactive diagnostic imaging agent according to the present invention, a radioactive fluorine-labeled amino acid compound serving as an effective component is first synthesized. The radioactive fluorine-labeled amino acid compound according to the present invention, that is, [$^{18}$F]-FACBC can be synthesized by the method disclosed in Goodman et al (Goodman et al., The Journal of Nuclear Medicine, (United States), 1999, 40, p.331-338).

For synthesis of anti-[$^{18}$F]-FACBC, a mixture comprising [$^{18}$F]fluoride ion, potassium carbonate and a phase transfer catalyst is first obtained. This mixture can be obtained by the conventional method (for example, a method described in the reference (Radioisotopes, 50, (2001), p.205-227, Radioisotopes, 50, (2001), p.228-256, "Production and quality control of radioactive agents for PET - Handbook of synthesis and clinical use- (2nd edition)" edited by PET Chemistry Workshop). [$^{18}$F]-labeling is conducted by adding, to this mixture, a solution of a labeling precursor 1-(N-(t-butoxycarbonyl)amino)-3-[((trifluoromethyl)sulfonyl) oxy]-cyclobutane-1-carboxylic acid ethylester in acetonitrile, and heating it to allow nucleophilic substitution reaction. This reaction solution is purified to remove the unreacted precursor, and then is given an acidic condition to conduct deprotection of amino groups. The resulting solution is neutralized and purified to obtain an anti-[$^{18}$F]-FACBC aqueous solution. Conditions in each step may be in accordance with conventional methods (for example, the method described in International Publication No. WO2004/056725 pamphlet).

The radioactive diagnostic imaging agent according to the present invention can be obtained by mixing a required amount of sugar lactone with the radioactive halogen-labeled organic compound obtained as above. The timing of mixing is not limited as long as the required amount of sugar lactose is contained in the final agent, but it is blended preferably before final adjustment operations such as radioactive concentration adjustment and pH adjustment are made for the radioactive diagnostic imaging agent.

The radioactive diagnostic imaging agent according to the present invention needs to have a radioactivity sufficient for PET imaging when it is used, and thus the radioactive concentration at the time of production is adjusted to meet that radioactivity. For example, the agent should have a radioactivity of 1.4GBq in about 2 mL and contain the above sugar lactone in a proportion of 10 μmol/mL immediately after production. Such a formulation of the agent provides a radioactivity of 50 to 225 MBq when the agent is used, and enables a sufficient PET imaging for adults. In addition, pH may be adjusted to a value acceptable for pharmaceuticals, for example, to 3-8.

EXAMPLE

Hereinafter, the present invention is described below in more detail by way of Examples. However, these Examples never limit the scope of the present invention.

(Comparative Example 1) Stability of anti-[$^{18}$F]-FACBC solution in the absence of additives

An anti-[$^{18}$F]-FACBC solution was prepared in accordance with the literature (Goodman et al., The Journal of Nuclear Medicine, (United States), 1999, 40, p.331-338). The obtained anti-[$^{18}$F]-FACBC solution was diluted with a physiological saline solution to prepare a sample solution (with diluting magnification of 4.7-7.0) so that it is supposed to have a radioactive concentration of 92.5 MBq/mL 6.5 hours after the initiation of production. In the present examples, examination was performed, supposing that 0 hour is the time when not only the preparation of the anti-[$^{18}$F]-FACBC solution but also the preparation of the solution into the sample solution were completed (hereinafter, referred to as sample solution preparation time). Since production time and sample solution preparation time were varied with different production yields, the radioactive concentration at the sample solution preparation time was, in fact, about 400-750 MBq/mL (details are shown in the table). Radioactivity on the sample solution was measured by using RADIOISOTOPE DOSE CALIBRATOR CAPINTEC MODEL CRC-15R (trade name; CAPINTEC, Inc.).

The obtained sample solution was sealed in a vital of 5mL in volume, analysis was performed by TLC analysis on the following conditions 0 hour and 9 hours after the sample solution preparation time, and radiochemical purity value was calculated in accordance with the following equation (1). Preparation of the sample solution and measurement of radiochemical purity were repeated 5 times, and the resulting value of radiochemical purity was used to evaluate stability.

TLC analysis conditions:

Mobile phase: acetonitrile/methanol/water/acetic acid = 20/5/5/1
TLC plate: Silica Gel 60F254 (trade name, thickness of membrane: 0.25 mm, manufactured by Merck & Co., Inc.)
Mobile length: 10 cm
TLC scanner: Rita Star (manufactured by Raytest)
Number of analysis: one or three times

$$\text{Radiochemical purity (\%)} = \frac{\text{Radioactivity of } [^{18}F]\text{FACBC peak}}{\text{Total radioactivity on TLC plate}} \times 100 \qquad (1)$$

The results are shown in Table 1. For all the sample solutions, the radiochemical purity of anti-[$^{18}$F]-FACBC solution was decreased, and the decrease (difference) of the radiochemical purity thereof was 1.57% in average. When data 0 hour and 9hours after the sample solution preparation time were subjected to Student's t test, the results was such that p-value was 0.001, t-value was 4.58, and even in case of 1.0% of significant level, decrease in radiochemical purity was significant. From the above, it was confirmed that the radiochemical purity of the anti-[$^{18}$F]-FACBC solution was decreased significantly until 9 hours after the preparation of the sample solution in the absence of additives.

Table 1: Change in radiochemical purity of *anti*-[$^{18}$F]-FACBC solution in the absence of additives

| Comparative Example | Range of radioactive concentration (MBq/mL) | Radiochemical purity (%) | | Difference* | t value | p value |
|---|---|---|---|---|---|---|
| | | 0 hour | 9 hours | | | |
| Comparative Example 1 | 401-754 | 98.15 ± 0.62 | 96.58 ± 0.56 | 1.57 | 4.58 | 0.0010 |
| *Difference = (radioichemical purity at 0 hour) - (radiochemical purity at 9 hours) | | | | | | |

<u>(Examples 1-3) Stability of anti-[$^{18}$F]-FACBC solution in the presence of ascorbic acid</u>

The anti-[$^{18}$F]-FACBC solution was prepared in the same manner as in Comparative Example 1, and diluted with the physiological saline solution under the same conditions as in Comparative Example 1.
Separately, 10, 100 and 200 μmol/mL ascorbic acid-containing physiological saline solutions were prepared, 50 μL of each of the ascorbic acid-containing physiological saline solutions was added to each 1mL of the above diluted anti-[$^{18}$F]-FACBC solution, to prepare, as sample solutions, anti-[$^{18}$F]-FACBC solutions containing ascorbic acid at concentrations of 0.5 (Example 1), 5.0 (Example 2) and 10.0 (Example 3) μmol/mL.

For each sample solution, radiochemical purity 0 hour and 9hours after the sample solution preparation time was measured under the same condition as in Comparative Example 1, and the obtained values of radiochemical purity were used to evaluate stability. Meanwhile, preparation and measurement of the sample solutions were repeated 3 times for Example 1, and 5 times for Examples 2 and 3.

The results are shown in Table 2. The decrease of radiochemical purity 9 hours after the sample solution preparation time was not more that 1% at all the concentrations of ascorbic acid. When data 0 hour and 9hours after the sample solution preparation time were subjected to Student's t test, the result was such that the changes of radiochemical purity were not significantly different at the significant level of 1% at all the concentrations of ascorbic acid. The above results indicated that ascorbic acid at concentrations of 0.5-10.0 μmol/mL can inhibit decomposition of the anti-[$^{18}$F]-FACBC solution.

Table 2: Change in radiochemical purity of *anti*-[$^{18}$F]-FACBC solution to which ascorbic acid is added

| | Range of radioactive concentration (MBq/mL) | Concentration of ascorbic acid (μmol/mL) | Radiochemical purity (%) | | Difference | t value | p value |
|---|---|---|---|---|---|---|---|
| | | | 0 hour | 9 hours | | | |
| Example 1 | 584-706 | 0.5 | 98.22 ± 0.17 | 97.78 ± 0.52 | 0.44 | 1.39 | 0.2357 |
| Example 2 | 401-754 | 5.0 | 98.29 ± 0.35 | 98.07 ± 0.47 | 0.22 | 0.85 | 0.4191 |
| Example 3 | 401-754 | 10.0 | 98.28 ± 0.37 | 98.13 ± 0.38 | 0.15 | 0.71 | 0.4963 |

As indicated in the above Examples, the radiolysis can be inhibited by adding a sugar lactone such as ascorbic acid to the radioactive fluorine-labeled amino acid compound such as anti-[$^{18}$F]-FACBC. Further, the radiolysis can be inhibited significantly at a concentration of not less than 0.5 μmol/mL. In addition, since the radiolysis was inhibited at

a concentration of 700 MBq/mL at maximum, it is considered that the radiolysis is naturally inhibited below that radioactive concentration, and thus a similar effect can be expected at 18.5 MBq/mL which is the minimum radioactive concentration in use.

INDUSTRIAL APPLICABILITY

The present invention can inhibit radiolysis of radioactive fluorine-labeled amino acid compounds which are useful as PET agents, and is useful in the field of radioactive pharmaceuticals.

## Claims

1. A radioactive diagnostic imaging agent, which comprises a sugar lactone and a radioactive fluorine-labeled amino acid compound represented by the following formula (1):

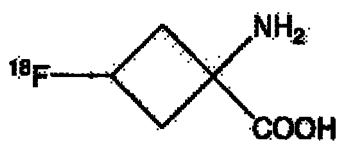

(1)

2. A radioactive diagnostic imaging agent according to claim 1, wherein the sugar lactone is selected from compounds that are acceptable at pharmaceutical additives.

3. A radioactive diagnostic imaging agent according to claim 1 or 2, wherein the sugar lactone is contained in an amount not less than 0.5 μmol/mL and not more than an amount acceptable for pharmaceutical additives.

4. A radioactive diagnostic imaging agent according to any one of claims 1 to 3, wherein the sugar lactone is ascorbic acid or glucono-o-lactone.

5. A radioactive diagnostic imaging agent according to any one of claims 1 to 4, which has a radioactive concentration of 25-125 MBq/mL when it is used.

**INTERNATIONAL SEARCH REPORT**

International application No. PCT/JP2007/067042

A. CLASSIFICATION OF SUBJECT MATTER

*A61K51/00*(2006.01)i, *A61K47/22*(2006.01)i, *G01T1/161*(2006.01)i

According to International Patent Classification (IPC) or to both national classification and IPC

B. FIELDS SEARCHED

Minimum documentation searched (classification system followed by classification symbols)
A61K51/00, A61K47/22, G01T1/161

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS(STN), CAplus(STN), EMBASE(STN), MEDLINE(STN), REGISTRY(STN), JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| Y | JP 2006-500319 A (MALLINCKRODT INC.), 05 January, 2006 (05.01.06), Full text; particularly, example 3 & EP 1356827 A1 & WO 2003/0090789 A1 & EP 1496946 A1 | 1-5 |
| Y | JP 2004-529884 A (BRISTOL-MYERS SQUIBB PHARMA CO.), 30 September, 2004 (30.09.04), Par. Nos. [0023], [0096] & US 2002/122769 A1 & WO 2002/067859 A2 & EP 1365813 A2 & US 6713042 B2 | 1-5 |

[X] Further documents are listed in the continuation of Box C. [ ] See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| | |
|---|---|
| Date of the actual completion of the international search<br>14 September, 2007 (14.09.07) | Date of mailing of the international search report<br>02 October, 2007 (02.10.07) |
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2007/067042

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2006-510706 A (GE HEALTHCARE LTD.), 30 March, 2006 (30.03.06), Par. Nos. [0026], [0027] & WO 2004/0056725 A1 & EP 1572601 A1 & US 2006/039855 A1 | 1-5 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03090789 A
- WO 04043497 A
- JP H10147542 B
- WO 2004056725 A


### Non-patent literature cited in the description


- *Pharmaceutical Affairs Bureau Notification No. 0624001,* 12
- **Goodman et al.** *The Journal of Nuclear Medicine, (United States),* 1999, vol. 40, 331-338
- *Radioisotopes,* 2001, vol. 50, 205-227
- *Radioisotopes,* 2001, vol. 50, 228-256
- Production and quality control of radioactive agents for PET - Handbook of synthesis and clinical use-(2nd edition)